# EUROPÄISCHE PATENTSCHRIFT

(11) **EP 1 658 023 B1**
(45) Veröffentlichungstag und Bekanntmachung des Hinweises auf die Patenterteilung: **21.03.2007**
(21) Anmeldenummer: 03818773.8
(22) Anmeldetag: 27.08.2003
(51) Int. Cl.: A61F 2/42, A61F 2/30

(54) **SPRUNGGELENK-ENDOPROTHESE**
ANKLE-JOINT ENDOPROSTHESIS
ENDOPROTHESE D'ARTICULATION DE CHEVILLE

(43) Veröffentlichungstag der Anmeldung: 24.05.2006
(73) Patentinhaber: WALDEMAR LINK GmbH & Co. KG, 22339 Hamburg (DE)
(72) Erfinder: KOFOED, Hakon, DK-2920 Charlottenlund (DK); KELLER, Arnold, 23863 Kayhude (DE); LINK, Helmut, D., 22397 Hamburg (DE)
(74) Vertreter: Glawe, Delfs, Moll
(86) Internationale Anmeldenummer: PCT/EP2003/009489
(87) Internationale Veröffentlichungsnummer: WO 2005/030098

(56) Entgegenhaltungen:
- EP-A- 1 097 680
- WO-A-01/89427
- WO-A-03/075802
- DE-U- 8 812 806
- DE-U- 20 010 346
- FR-A- 2 676 917
- US-A- 5 019 103
- US-A- 5 152 797
- US-A- 5 458 637

## Beschreibung

Zum Ersatz des Sprunggelenks ist eine Endoprothese bekannt, die aus einer mit dem Sprungbein zu verbindenden Komponente, einer mit dem Schienbein zu verbindenden Komponente und einem Zwischenteil besteht (DE-U-88 12 806, Prospekt "LINK S.T.A.R. Totale Sprunggelenkprothese [H. Kofoed] der Firma Waldemar Link (GmbH & Co.), Hamburg). Die Sprungbeinkomponente und der Zwischenteil wirken über Gleitflächen zusammen, die Beugung und Streckung in der Sagittalebene ermöglichen. Die Schienbeinkomponente und der Zwischenteil bilden zusammenwirkende Gleitflächen, die eine Rotation um die Hochachse erlauben. Sie können eben ausgeführt sein, um Ausgleichsbewegungen in AP- und LM-Richtung zu ermöglichen. Die Stabilisierung geschieht durch den natürlichen Bandapparat.

Die ober- und unterseitigen Gleitflächen des Zwischenteils sind bei der bekannten Prothese in der Frontalebene parallel zueinander orientiert weil der prothetische Ersatz des Gelenks nicht zu einer Richtungsänderung führen soll. Jedoch hat sich gezeigt, daß die lateralen und medialen Bänder des Gelenks nach der Operation nicht selten eine unterschiedliche Spannung aufweisen, woraus Beschwerden resultieren können. Dies kann auf Unregelmäßigkeiten der Anatomie beruhen oder darauf, daß der Operateur eine ungünstige Ausrichtung der am Schienbein zur Verbindung mit der Prothese vorgesehenen Resektionsfläche gewählt hat.

Der Erfindung liegt die Aufgabe zugrunde, die Unausgewogenheit zwischen den anatomischen bzw. operativen Gegebenheiten einerseits und der Prothese andererseits zu vermeiden oder zu lindern.

Die erfindungsgemäße Lösung besteht in den Merkmalen des Anspruchs 1 bzw. denen des Anspruchs 6. Danach ist vorgesehen, daß der Zwischenteil und/oder die Schienbeinkomponente keilförmig ausgebildet sind. Stellt der Operateur fest, daß bei Verwendung einer normalen Prothese die Bandspannung unterschiedlich ist, kann er dies durch Verwendung von Korrekturkomponenten, die in der Frontalebene eine Keilform haben, ausgleichen. Die dickere Seite der keilförmigen Korrekturplatte wird auf der Seite angeordnet, auf der bei Verwendung von Normalkomponenten die Bandspannnung unzureichend wäre. Wenn er erkennt, daß die tibiale Resektionsfläche nicht lotrecht zur Tibiarichtung verläuft oder wenn er aus anderen Gründen wünscht, daß die Prothesenebenen nicht lotrecht zur Tibiaachse verlaufen, kann er auch Korrekturkomponenten einsetzen, deren Keilform in der Sagittalebene liegt. Wenn es dem Operateur darum geht, daß die untere Gleitfläche der oberen Komponente eine bestimmte Ausrichtung gegenüber der Tibiarichtung oder der Belastungsrichtung hat, wird er im allgemeinen eine als Korrekturkomponente ausgebildete Schienbeinkomponente verwenden. Wenn hingegen die Ausrichtung der tibialen Resektionsfläche richtig ist und es darum geht, der Anatomie des Fußes Rechnung zu tragen, wird er einen als Korrekturkomponente ausgebildeten Zwischenteil bevorzugen.

Stand der Technik unter Art. 54(3) EPU ist die keilförmige Ausbildung des Zwischenteils in der Frontalebene, entsprechend dem Gegenstand der älteren, nicht vorveröffentlichten Patentanmeldung PCT/EP02/02573.

Die Feststellung, daß die obere Komponente keilförmig ist, macht in der Praxis keine Schwierigkeiten, da sowohl ihre oberseitige Verbindungsfläche als auch ihre unterseitige Gleitfläche eben oder im wesentlichen eben sind. Für die Bestimmung einer etwaigen Keilform des Zwischenteils in der Sagittalebene ist der Vergleich mit den normalen Zwischenteilen maßgebend. Im übrigen läßt sich nicht nur die Richtung der etwa ebenen oberseitigen Gleitfläche des Zwischenteils leicht bestimmen sondern auch die Gesamtausrichtung der unterseitigen Gleitfläche. Keilförmig im Sinne der vorliegenden Erfindung ist ein Zwischenteil in der Sagittalebene dann, wenn er sich im Vergleich mit dem normalen Zwischenteil nach anterior oder posterior verdickt.

Eine Keilform in der Sagittalebene kann im Rahmen der vorliegenden Erfindung mit einer Keilform in der Frontalebene kombiniert sein. Jedoch können die Keilform in der Sagittal- oder Frontalebene auch ohne Keilform in der Frontal- bzw. Sagittalebene auftreten.

Der Keilwinkel liegt zweckmäßigerweise zwischen 1° und 16°, vorzugsweise zwischen 3° und 8°.

Damit sich die Ausrichtung der Keilform des Zwischenteils nicht durch Verdrehung des Zwischenteils um die Hochachse verändern kann, wird seine Ausrichtung zweckmäßigerweise zwangsweise vom Sprungbein oder vom Schienbein her festgelegt, indem die zusammenwirkenden Gleitflächenpaare entsprechend richtungsbestimmend (beispielsweise zylindrisch) ausgebildet sind. Dazu ist besonders das Gelenk zwischen dem Sprungbein und dem Zwischenteil geeignet.

Damit der Operateur unter unterschiedlichen Keilwinkeln auswählen kann, müssen ihm zu jeder Prothese mehrere unterschiedliche Korrekturkomponenten zur Verfügung stehen. Um den damit verbundenen Aufwand zu verringern, ist es zweckmäßig, die Korrekturkomponenten so einfach wie möglich zu gestalten. Es kann deshalb zweckmäßig sein, sie aus einem stets gleichbleibenden Standardteil und einem Keilteil zusammenzusetzen. Der Standardteil wird in nur einfacher Ausfertigung vorgesehen. Nur die Keilteile brauchen in unterschiedlichen Varianten vorhanden zu sein. Das gilt insbesondere dann, wenn die obere Komponente als Korrekturkomponente verwendet wird und der Standardteil die Einrichtungen bildet, die zu Befestigung am Schienbein erforderlich sind.

Die Erfindung wird im folgenden näher unter Bezugnahme auf die Zeichnung erläutert, die vorteilhafte Ausführungsbeispiele veranschaulicht. Es zeigen:
- Fig. 1: einen Sagittalschnitt durch ein mit der Prothese versorgtes Gelenk,
- Fig. 2: die Prothese perspektivisch aufgefächert,
- Fig. 3: einen perspektivischen Blick in die Anordnung der implantierten oberen und unteren Prothesenkomponenten vor dem Einsetzen des Zwischenteils,
- Fig. 4: einen Frontalschnitt durch eine obere Korrekturkomponente,
- Fig. 5: einen Sagittalschnitt durch eine obere Korrekturkomponente,
- Fig. 6: einen Sagittalschnitt durch einen Korrektur-Zwischenteil, und
- Fig. 7: eine obere Korrekturkomponente, die zweiteilig ausgebildet ist.

Zwischen dem Schienbein 1 und dem Sprungbein 2 ist die Prothese anzuordnen, die aus der oberen Komponente 3, der unteren Komponente 4 und dem Zwischenteil 5 besteht. Die obere Komponente 3 weist einen plattenförmigen Teil 6 auf, dessen Unterseite 7 eine ebene Gleitfläche bildet. Vorsprünge 8 dienen zur Befestigung in entsprechenden Resektionsausnehmungen 9 des Schienbeins 1.

Die untere Komponente 4 bildet eine konvex gewölbte Gleitfläche 10, die zylindrisch oder kegelig ausgebildet sein kann. Sie trägt eine Rippe 11, die in der Richtung der Relativbewegung des Zwischenteils bei der Beuge- und Streckbewegung liegt. Ferner weist die untere Komponente seitliche Facetten 12 zum Zusammenwirken mit entsprechenden Gleitflächen des Schienbeins 1 und des Wadenbeins 13 auf.

Der Zwischenteil 5 besitzt eine ebene, zur Gleitfläche 7 passende Oberseite 15 und eine unterseitige Gleitfläche 16, die komplementär zur Gleitfläche 10 der unteren Komponente 4 ausgebildet ist. Sie enthält eine Nut 17 zur Aufnahme der Rippe 11. Dadurch wird der Zwischenteil 5 seitlich im Verhältnis zur unteren Komponente 4 geführt. Nur Beuge- und Streckbewegungen sind ihr erlaubt.

Die oberen und unteren Komponenten 3 und 4 bestehen zweckmäßigerweise aus Metall und der Zwischenteil 5 aus gleitgünstigem Kunststoff wie Polyethylen. Jedoch sind auch andere Werkstoffe mit hinreichender Festigkeit und Gleitfähigkeit verwendbar, beispielsweise Keramik.

Infolge der komplementären Form der Gleitflächen 10 und 16 sowie durch die Rippe 11 im Zusammenwirken mit der Nut 17 ist der Zwischenteil 5 bezüglich der Hochachse gegenüber der Sprunggelenkkomponente 4 nicht drehbar. Seine Ausrichtung ist also durch diejenige der unteren Komponente festgelegt. Während die gezeigte Ausführungsform derartige Rotationsbewegungen zwischen der unteren Komponente und dem Zwischenteil um die Hochachse vollständig ausschließt, sind auch Ausführungen denkbar, in denen sie in vorbestimmten Grenzen erlaubt oder durch die Ausbildung der Gleitflächen lediglich gehemmt oder nicht ausgeschlossen sind.

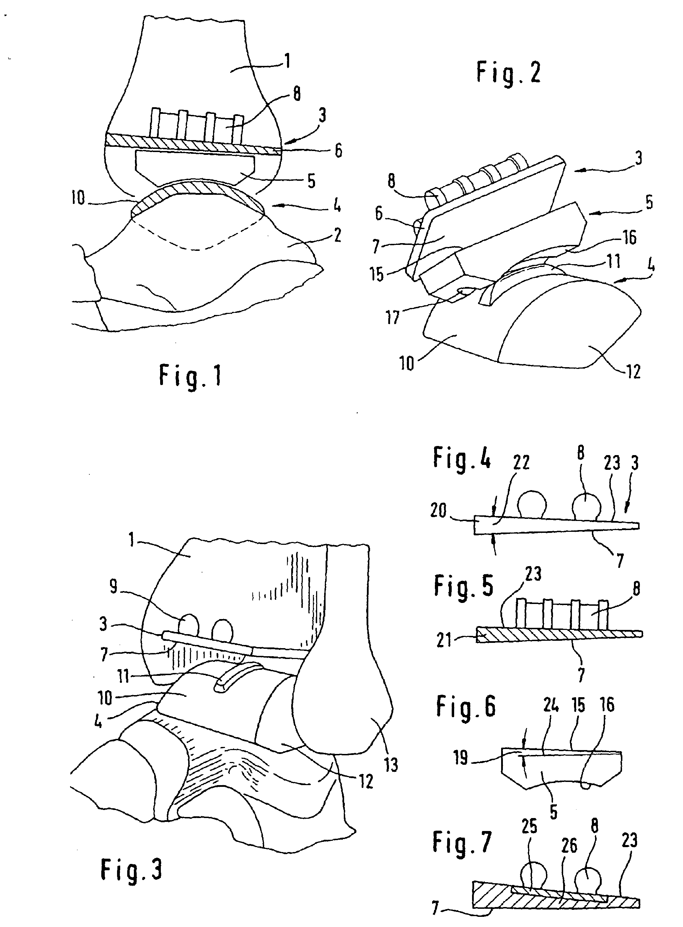

Die vorstehende Erläuterung anhand von Fig. 1 bis 3 gilt sowohl für Ausführungen mit Normalkomponenten als auch für Ausführungen mit Korrekturkomponenten.

In Fig. 4 bis 6 sind Beispiele für Korrekturkomponenten gezeigt. Fig. 4 zeigt eine Frontalansicht einer als Korrekturkomponente ausgebildeten oberen Komponente 3. Nahe dem in der Zeichnung links erscheinenden Rand 20 ist sie dicker als am gegenüber liegenden Rand ausgebildet. Die Komponente ist in bezug auf ihre frontale Mittelebene symmetrisch ausgebildet, so daß die verdickte Seite 20 nach Wahl des Operateurs auf der lateralen oder medialen Seite des Gelenks liegen darf.

Fig. 5 zeigt einen Sagittalschnitt durch die obere Komponente 3. Sie ist an dem in der Zeichnung links erscheinendem Ende 21 keilförmig verdickt ausgebildet. Die Oberseite dieser Komponente ist symmetrisch in bezug auf die Frontalebene ausgebildet. Daher kann das verdickte Ende nach Wahl des Operateurs vorne oder hinten im Gelenk angeordnet sein. Der Keilwinkel 22 zwischen der oberen Befestigungsfläche 23 und der unteren Gleitfläche 7 ist in beiden Beispielen in der Größenordnung von 5° dargestellt.

Fig. 6 zeigt einen Sagittalschnitt durch einen Korrektur-Zwischenteil 5. Seine als Gleitfläche ausgebildete Unterseite 16 hat eine Gesamtausrichtung, die etwa parallel zu der Hilfslinie 24 verläuft, die zur Darstellung des Keilwinkels 19 nahe der oberen Gleitfläche 15 eingezeichnet ist. In diesem Fall ist vorausgesetzt, daß die Linie 24 bei den Normal-Zwischenteilen parallel zur Gesamtrichtung der Unterfläche 16 verläuft. Maßgebend für die Feststellung einer Keilform des Zwischenteils ist stets der Vergleich mit den Normalkomponenten des Prothesensystems.

Es ist nicht erforderlich, daß sich eine Korrektur auf nur jeweils eine Komponente beschränkt. Vielmehr können sowohl für die obere Komponente als auch für den Zwischenteil Korrekturkomponenten verwendet werden. Diese Möglichkeit zeigt Fig. 1.

Sobald der Operateur die untere Komponente 4 implantiert hat, kann er mittels geeigneter Instrumente bei gespannten Seitenbändern feststellen, ob die Resektionsfläche 25 des Schienbeins gegenüber der unteren Komponente 4 einen normalen Verlauf hat oder ob eine Korrektur notwendig ist. Im letzteren Fall entscheidet er, ob für die obere Komponente oder den Zwischenteil oder beide eine Korrekturkomponente gewählt werden soll und wie stark und in welcher Richtung die jewilige Keilform sein soll. Eine entsprechende Messung ist auch dann noch möglich, wenn die obere Komponente 3 eingesetzt ist. Danach kann entschieden werden, ob als Zwischenteil eine Korrekturkomponente verwendet wird.

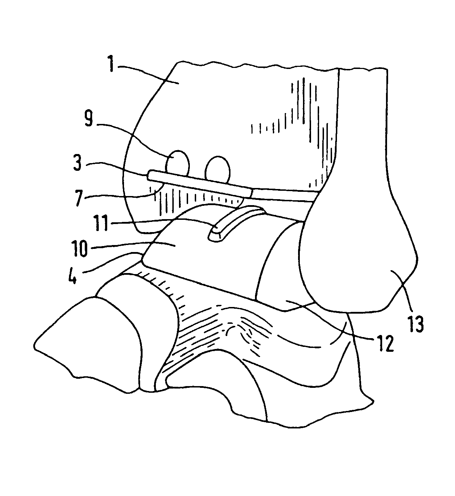

Fig. 7 veranschaulicht die Zusammensetzung der oberen Komponente 3 aus einem Standardteil 25 und einem Keilteil 26. Da der Standardteil 25 die Befestigungsorgane 8 bildet, kann der Keilteil 26, von dem mehrere Exemplare mit unterschiedlichen Keilwinkeln zur Verfügung stehen, entsprechend einfacher ausgebildet sein. Die beiden Teile können in beliebiger, bekannter Weise miteinander verbunden werden. Beispielsweise können sie miteinander verschraubt werden. Sie können auch mit komplementären, ineinander greifenden Vorsprüngen und Ausnehmungen versehen sein, die es gestatten, die beiden Teile ohne wesentlichen Aufwand zusammenzustecken.

## Patentansprüche

1. Endoprothese zum Ersatz eines Sprunggelenks, bestehend aus einer unteren mit dem Sprungbein (2) zu verbindenden Komponente (4), die eine oberseitige Gleitfläche (10) bildet, einer oberen Komponente (3), die eine unterseitige Gleitfläche (7) bildet und zur Verbindung mit einer Resektionsfläche (25) des Schienbeins eine obere Verbindungsfläche (23) aufweist und aus einem Zwischenteil, der zwei mit den Gleitflächen (7, 10) der oberen und unteren Komponenten (3, 4) zusammenwirkende Gleitflächen (15, 16) aufweist, wobei die obere Komponente (3) im Frontal- und/oder Sagittalschnitt zwischen ihrer unterseitigen Gleitfläche (7) und ihrer oberseitigen verbindungsfläche (23) und/oder der zwischenteil (5) zwischen seiner Gleitfläche an der Oberseite (15) und 9 der Gesamtausrichtung der Gleitfläche an der Unterseite (16) im Sagittalschnitt keilförmig sind.

2. Endoprothese nach Anspruch 1 **dadurch gekennzeichnet, daß** die an der unteren Komponente (4) und dem Zwischenteil (5) zusammenwirkenden Gleitflächen (10, 16) im wesentlichen unrotierbar bezüglich der Hochachse zusammenwirken.

3. Endoprothese nach Anspruch 1 **dadurch gekennzeichnet, daß** die an der oberen Komponente (4) und dem Zwischenteil (5) zusammenwirkenden Gleitflächen (7, 15) bezüglich der Hochachse rotierbar zusammenwirken.

4. Endoprothese nach einem der Ansprüche 1 bis 3 **dadurch gekennzeichnet, daß** der Keilwinkel (19, 22) zwischen 1° und 16° liegt.

5. Endoprothese nach einem der Ansprüche 1 bis 4, **dadurch gekennzeichnet, daß** die keilförmige Komponente (3) sich aus einem Keilteil (26), der mit unterschiedlichem Keilwinkel zur Verfügung steht, und einem Standardteil (25) zusammensetzt.

6. System von Endoprothesen zum Ersatz des Sprunggelenks, die aus einer unteren, mit dem Sprungbein (2) zu verbindenden Komponente (4), die eine oberseitige Gleitfläche (10) bildet, einer oberen Komponente (3), die eine unterseitige Gleitfläche (7) bildet und zur Verbindung mit einer Resektionsfläche (25) des Schienbeins (1) eine Verbindungsfläche (23) aufweist, und einem Zwischenteil (5), der zwei mit den Gleitflächen (7, 10) der oberen und unteren Komponenten (3, 4) zusammenwirkende Gleitflächen (15, 16) aufweist, bestehen, wobei das System normale obere Komponenten und Zwischenteile umfaßt, deren Ober- und Unterseite einen im wesentlichen parallelen Gesamtverlauf haben, wobei es gegen die normalen oberen Komponenten (3) austauschbare Korrekturkomponenten umfaßt, die zwischen ihren Ober- und Unterseiten (7, 23) in der Sagittalebene und/oder Frontalebene keilförmig geformt sind und/oder gegen die normalen Zwischenteile (5) austauschbare Korrektur-Zwischenteile umfaßt, die zwischen ihrer Gleitfläche an der Oberseite (15) und dem Gesamtverlauf der Gleitfläche an der Unterseite (16) in der Sagittalebene im Vergleich mit den normalen Zwischenteilen (5) keilförmig sind.

## Claims

1. Endoprosthesis for replacing an ankle joint, comprising a lower component (4) which is to be connected to the ankle bone (2) and which forms a top slide surface (10), an upper component (3) which forms a bottom slide surface (7) and which has an upper connection surface (23) for connection to a resection surface (25) of the shin bone, and an intermediate part which has two slide surfaces (15, 16) interacting with the slide surfaces (7, 10) of the upper and lower components (3, 4), the upper component (3) being wedge-shaped in frontal and/or sagittal section between its bottom slide surface (7) and its top connection surface (23) and/or the intermediate part (5) being wedge-shaped in sagittal section between its slide surface at the top face (15) and the overall orientation of the slide surface at the bottom face (16)

2. Endoprosthesis according to Claim 1, **characterized in that** the interacting slide surfaces (10, 16) on the lower component (4) and the intermediate part (5) interact substantially nonrotatably with respect to the vertical axis.

3. Endoprosthesis as claimed in Claim 1, **characterized in that** the interacting slide surfaces (7, 15) on the upper component (4) and the intermediate part (5) interact rotatably with respect to the vertical axis.

4. Endoprosthesis as claimed in one of Claims 1 to 3, **characterized in that** the wedge angle (19, 22) is between 1° and 16.

5. Endoprosthesis as claimed in one of Claims 1 to 4, **characterized in that** the wedge-shaped component (3) is made up of a wedge part (26), available with a varying wedge angle, and of a standard part (25).

6. System of endoprostheses for replacing the ankle joint, comprising a lower component (4) which is to be connected to the ankle bone (2) and which forms a top slide surface (10), an upper component (3) which forms a bottom slide surface (7) and which has a connection surface (23) for connection to a resection surface (25) of the shin bone (1), and an intermediate part (5) which has two slide surfaces (15, 16) interacting with the slide surfaces (7, 10) of the upper and lower components (3, 4), the system including normal upper components and intermediate parts whose top face and bottom face have a substantially parallel overall course, it including corrective components which can be used in exchange for the normal upper components (3) and which are wedge-shaped in the sagittal plane and/or frontal plane between their top and bottom faces (7, 23) and/or corrective intermediate parts which can be used in exchange for the normal intermediate parts (5) and which, between their slide surface at the top face (15) and the overall course of the slide surface at the bottom face (16), are wedge-shaped in the sagittal plane compared to the normal intermediate parts (5).

## Revendications

1. Endoprothèse pour le remplacement d'une articulation sous-astragalienne, consistant en un composant inférieur (4) à relier à l'astragale (2) et qui constitue une surface de glissement supérieure (10), en un composant supérieur (3) qui constitue une surface de glissement inférieure (7) et présente, pour la connexion à une surface de résection (25) du tibia, une surface de connexion supérieure (23) et en une pièce intermédiaire qui présente deux surfaces de glissement (15, 16) coopérant avec les surfaces de glissement (7, 10) des composants supérieur et inférieur (3, 4), le composant supérieur (3) étant en forme de coin en coupe frontale et/ou sagittale entre sa surface de glissement inférieure (7) et sa surface de connexion supérieure (23) et/ou la pièce intermédiaire (5) l'étant entre sa surface de glissement en coupe sagittale sur la surface (15) et dans l'orientation globale de la surface de glissement sur la face inférieure (16).

2. Endoprothèse selon la revendication 1, **caractérisée en ce que** les surfaces de glissement (10, 16) coopérant au niveau du composant inférieur (4) et de la pièce intermédiaire (5) coopèrent sensiblement sans pouvoir tourner par rapport à l'axe vertical.

3. Endoprothèse selon la revendication 1, **caractérisée en ce que** les surfaces de glissement (7, 15) coopérant au niveau du composant supérieur (4) et de la pièce intermédiaire (5) coopèrent en pouvant tourner par rapport à l'axe vertical

4. Endoprothèse selon l'une des revendications 1 à 3, **caractérisée en ce que** l'angle de bec (19, 22) est compris entre 1° et 16°.

5. Endoprothèse selon l'une des revendications 1 à 4, **caractérisée en ce que** le composant en forme de coin (3) est composé d'une pièce de coin (26) qui est disponible avec un angle de bec différent et d'une pièce standard (25).

6. Système d'endoprothèses pour le remplacement de l'articulation sous-astragalienne qui consistent en un composant inférieur (4) à relier à l'astragale (2) et qui constitue une surface de glissement supérieure (10), un composant supérieur (3), qui constitue une surface de glissement inférieure (7) et présente, pour la connexion à une surface de résection (25) du tibia (1), une surface de connexion (23), et en une pièce intermédiaire (5) qui présente deux surfaces de glissement (15, 16) coopérant avec les surfaces de glissement (7, 10) des composants supérieur et inférieur (3, 4), le système englobant des pièces intermédiaires et composants supérieurs normaux dont les faces supérieure et inférieure ont une extension globale sensiblement parallèle, ce système incluant des composants de correction échangeables contre les composants supérieurs normaux (3) et qui sont conformés en forme de coin entre ses faces supérieure et inférieure (7, 23) dans le plan sagittal et/ou frontal et/ou des pièces intermédiaires de correction échangeables contre les pièces intermédiaires normales (5) qui, entre leur surface de glissement sur la face supérieure (15) et l'extension globale de la surface de glissement sur la face inférieure (16), sont en forme de coin dans le plan sagittal comparativement aux pièces intermédiaires normales (5).
